## Europäisches Patentamt
## European Patent Office
## Office européen des brevets

(19)

(11) Veröffentlichungsnummer: **0 144 714**
**A2**

# (12) EUROPÄISCHE PATENTANMELDUNG

(21) Anmeldenummer: **84113093.3**

(22) Anmeldetag: **31.10.84**

(51) Int. Cl.⁴: **C 07 K 3/02**
**C 07 K 3/24, A 61 K 37/02**
**A 61 K 35/16**

---

(30) Priorität: **09.12.83 DE 3344656**

(43) Veröffentlichungstag der Anmeldung:
**19.06.85 Patentblatt 85/25**

(84) Benannte Vertragsstaaten:
**AT BE CH DE FR GB IT LI LU SE**

(71) Anmelder: **SCHWAB & CO. GES.M.B.H.**
**Doerenkampgasse 4**
**A-1100 Wien(AT)**

(84) Benannte Vertragsstaaten:
**BE CH FR GB IT LI LU SE AT**

(71) Anmelder: **Lentia Gesellschaft mit beschränkter Haftung**
**Arabellastrasse 4 Postfach 81 05 08**
**D-8000 München 81(DE)**

(84) Benannte Vertragsstaaten:
**DE**

(72) Erfinder: **Kaltschmid, Helmut, Dr.**
**Dammgasse 11**
**A-2540 Bad Vöslau(AT)**

(72) Erfinder: **Wahl, Heinrich**
**Am Waldrand 10**
**A-2500 Sooss(AT)**

(74) Vertreter: **Allgeuer, Dorothea et al,**
**Chemie Linz AG Patentabteilung St. Peter-Strasse 25**
**A-4020 Linz(AT)**

---

(54) Verfahren zur Herstellung einer Serumproteinlösung.

(57) Herstellung einer Serumproteinlösung mit gegenüber natürlichen Blutserum unveränderter Antikomplementäraktivität ausgehend von Cohn-Fraktion IV - 1 und V als Albuminfraktion sowie Cohn II und III-Niederschlag als Globulinfraktion, wobei beide getrennt durch eine fraktionierte Ammonsulfatfällung gereinigt wurden, der bei der Globulinfraktion eine Wärmebehandlung bei 45 - 65°C zur gezielten Aggregatbildung zuvor aggregierter Anteile vorangeht. Die gereinigte Albuminfraktion wird pasteurisiert, bevor sowohl diese als auch das gereinigte Globulin in isotone Lösungen gleichen oder annähernd gleichen Proteingehaltes übergeführt und im gewünschten Verhältnis gemischt werden.

EP 0 144 714 A2

Verfahren zur Herstellung
einer Serumproteinlösung

Die vorliegende Erfindung betrifft ein Verfahren zur Herstellung einer Serumproteinlösung mit gegenüber natürlichem Blutserum unveränderter Antikomplementäraktivität, die für die intravenöse Verabreichung als Plasmasubstitut geeignet ist.

Serumeiweißpräparate sind wertvolle Blutderivate, die als Plasmasubstitut verabreicht werden können. Dabei wirkt nicht nur das darin enthaltene Albumin als Transportprotein, sondern es ist auch der Globulin-Gehalt solcher Präparate, vor allem jener an Gamma-Globulinen von Bedeutung, da die durch diese Stoffe hervorgerufene passiv-Immunisierung den durch Blutverlust geschwächten Körper unterstützt, bzw. die Immunitätslage des Patienten verbessert.

Sehr wünschenswert wäre bei geschwächten Patienten die Gabe von Immunglobulin M, dessen Bedeutung in den hohen Antikörpertitern gegen gramnegative Bakterien liegt. Seine Verabreichung in Form eines intravenös verträglichen Gammaglobulinpräparates ist jedoch bis heute noch nicht möglich. Wohl aber gelingt dessen iv-Applikation bei Gabe von Humanserum bzw. Humanserumpräparationen. Solche Präparationen weisen ferner einen Gehalt an alpha und beta-Globulinen auf, deren Wirkung zwar nicht voll geklärt ist, von denen aber ebenfalls eine positive Wirkung zu vermuten ist.

Der einfachste Weg, eine solche humane Serumpräparation zu erhalten, ist die Gewinnung von gepooltem Humanserum, das sterilfiltriert und verfüllt wird. Der Nachteil solcher Präparationen liegt darin, daß sie einer Sterilisation durch Erhitzen oder Pasteurisieren nicht zugänglich sind, sodaß ein gewisses Infektionsrisiko nicht auszuschließen ist.

Es wurde daher vorgeschlagen, (siehe W.Stephan, "Hepatitis-Free and Stable Human Serum for intravenous Therapy", Vox Sanguin. 20, S.442 bis 457) zur Herstellung von solchen Serumpräparationen aus Spenderblut zunächst die festen Bestandteile (rote Blutkörperchen, Blutplättchen) abzutrennen, durch eine Aerosil-Behandlung bei 45°C die stark zur Denaturierung neigenden Lipoproteine zu binden und abzutrennen und das überstehende Serumpräparat nach Zugabe von beta-Propiolacton bei 5°C durch UV-Licht-Bestrahlung zu sterilisieren. Diese Behandlung führt jedoch zu einer Acylierung und damit Veränderung von Proteinen, die bei Applikation Nebenwirkungen verursachen und die biologische Wirkung einschränken kann.

Während in der genannten Veröffentlichung in Vox Sanguinis von Humanserum ausgegangen wird, das ohne Auftrennung der Reinigung und Sterilisation unterworfen wird, wird gemäß AT-PS 367.297 vorgeschlagen, zur Herstellung von Serumeiweißpräparaten gezielt von Eiweißfraktionen auszugehen, die nach dem Fraktionierverfahren nach Cohn erhalten wurden. Diese Fraktionen, bei denen es sich um die Cohn Fraktionen I - IV handelt, werden gemischt, wobei ein gegenüber dem Humanblut erhöhter Globulinanteil gewählt wird, in eine isotone Lösung übergeführt, stabilisiert, gereinigt und gegebenenfalls sterilisiert. Speziell verwendet wird dazu die Cohn Fraktion IV, welche in erster Linie Albumin neben alpha- und beta-Globulinen enthält, sowie die Subfraktionen III1 und III2, die nach Auflösen der die gamma-Globuline enthaltenden Cohn Fraktion II/III durch Behandlung mit Polyäthylenglykol erhalten werden und neben den gamma-Globulinen auch etwas Albumin sowie alpha-und beta-Globuline enthalten. Die Reinigung der gemeinsamen Auflösung dieser drei Fraktionen besteht dann in einer Abtrennung jener Bestandteile, die in der zur Auflösung verwendeten Kochsalzlösung nicht löslich waren, einer Behandlung mit kolloidaler Kieselsäure, einer Klärfiltration und einer Dialyse, wobei in letztgenanntem Schritt eine Aufkonzentrierung auf eine Gesamteiweißkonzentration von 3,4 bis 4,5 Gew.% erfolgt. Eine Sterilisierung des so erhaltenen

C144714

Präparates wird in der genannten AT-PS an sich nicht für notwendig gehalten. Es wird aber festgestellt, daß eine solche sowohl über eine Pasteurisierung (10 Stunden bei 60°C) als auch durch UV-Bestrahlung in Gegenwart von beta-Propiolacton erzielbar sei. Wird eine Pasteurisation jedoch durchgeführt, was in jedem Falle wünschenswert wäre, ist mit einer hohen antikoplementären Aktivität zu rechnen, die unangenehme Nebenerscheinungen bewirken kann. Diese antikomplementäre Aktivität ist darauf zurückzuführen, daß die Globulin-Fraktionen durch die Cohn-Fraktionierung teilweise aggregiert worden sind, diese Aggregate bei der nachfolgenden adsorptiven Reinigung nicht entfernt werden und beim Erhitzen dann weitere Aggregation erfolgt. Wird hingegen als Methode der Sterilisierung das beta-Propiolactonverfahren gewählt, sind durch die dabei auftretende Derivatisierung von Proteinen diese gegenüber nativen Proteinen verändert, was sich auch auf deren Antikörperaktivität auswirken kann.

Der vorliegenden Erfindung liegt daher die Aufgabe zugrunde, eine Serumpräparation zu schaffen, bei der bei möglichster Virus- bzw. Bakterienfreiheit weder der Nachteil einer erhöhten Antikomplementäraktivität, noch der einer möglichen Derivatisierung von Proteinen in Kauf genommen werden muß, sodaß die Proteine möglichst nativ bleiben.

Diese Aufgabe konnte erfindungsgemäß dadurch gelöst werden, daß zur Herstellung der Serumpräparation zwar ebenfalls wie in der AT-PS 367.297 von einer Fraktion nach Cohn ausgegangen wird, daß jedoch vor der nachfolgenden Mischung die Albuminfraktion über eine Ammonsulfatfällung gereinigt und pasteurisiert sowie die Globulinfraktion zur gezielten Entfernung der gebildeten Aggregate einer Wärmebehandlung bei 45 - 65°C und einer nachfolgenden fraktionierten Fällung mit Ammonsulfat unterzogen wird. Dadurch wird nicht nur erreicht, daß der Immunglobulinanteil möglichst nativ bleibt, sondern derart gereinigte Immunglobuline gelten ebenfalls als hepatitis-sicher.

Gegenstand der vorliegenden Erfindung ist demnach ein Verfahren zur Herstellung einer Serumproteinlösung mit gegenüber natürlichem Blutserum unveränderter Antikomplementäraktivität die für die intravenöse Verabreichung als Plasmasubstitut geeignet ist, durch Fraktionierung von Humanplasma oder Humanserum mittels Äthanol nach Cohn, sowie Mischen und Auflösen von dabei erhaltenen, gegebenenfalls weiter gereinigten Albumin- und Globulinfraktionen in isotoner Kochsalzlösung, dadurch gekennzeichnet, daß sowohl die bei der Cohn-Fraktionierung erhaltene, als Fraktion IV - 1 und V bezeichnete Albuminfraktion, als auch die als Cohn II und III-Niederschlag bezeichnete Globulinfraktion getrennt einer Reinigung mittels Ammonsulfatfällung unter zogen werden, wobei die Globulinfraktion vor der Fällung mit Ammonsulfat in Form einer wäßrigen Lösung bei neutralem pH durch eine Wärmebehandlung bei 45 - 65°C einer gezielten Aggregatbildung zuvor aggregierter Anteile sowie die gereinigte Albuminfraktion einer Pasteurisierung unterworfen wird, bevor sie mittels Wasser und Kochsalz in zwei getrennte isotone Lösungen gleichen oder annähernd gleichen Proteingehaltes übergeführt, im gewünschten Verhältnis gemischt und in üblicher Weise durch Klär- und Sterilfiltration in haltbare Form übergeführt werden.

Diese so erhaltene, erfindungsgemäße Serumproteinlösung kann von der Herstellung her als virus- und bakterienfrei bezeichnet werden.

Zweckmäßigerweise wird die Menge der zur Auflösung dienenden Kochsalzlösung so gewählt, daß ein Proteingehalt der beiden Fraktionen und somit der durch Vereinigung derselben entstandenen Serumproteinlösung von 4,75 - 5,25 Gew.% resultiert. Der Kochsalzgehalt soll hiebei bei 130 - 160 m Mol NaCl/l liegen. Solche Lösungen sind isoton. Die Menge an Albuminfraktion und Globulinfraktion wird vorzugsweise so gewählt, daß etwa ein Verhältnis von Albumin zu Globulin erhalten wird, das dem im humanen Serum entspricht. Das dazu nötige Mischungsverhältnis von Albuminlösung und Globulinlösung etwa gleicher Konzentration beträgt dazu etwa 4 : 1, woraus in der Regel ein Gehalt von etwa 65 - 75 Gew.% Albumin und 10 - 16 Gew.% gamma-Globulin, bezogen auf den Proteingehalt des Präparates, resultiert. Das erfindungsgemäße Verfahren ist aber mit besonderem Vorteil auch auf die Herstellung von Serumpräparationen mit gegenüber dem humanem Serum erhöhtem gamma-Globulinanteil anwendbar. In diesem Fall ist die erfindungsgemäße gezielte weitere Aggregation von vorher gebildeten Aggregaten durch die Wärmebehandlung von besonderer Bedeutung, da die Gefahr des Auftretens von Antikomplementäraktivität mit steigendem gamma-Globulingehalt zunimmt und der Schutz vor dieser Nebenwirkung durch den Albumingehalt mit dessen gewichtsmäßiger Abnahme geringer wird. Speziell trifft dieses Problem dann zu, wenn ein höherer Ig-M-Anteil vorgesehen ist.

Für die praktische Durchführung des erfindungsgemäßen Verfahrens wird Humanplasma oder Humanserum nach der Methode von Cohn, die in der AT-PS 367.297 ausführlich dargestellt ist, in Fraktionen zerlegt. Von diesen Fraktionen dient erfindungsgemäß als Albuminquelle Cohn IV - 1 + V-Niederschlag, als Globulinquelle Cohn II/III-Niederschlag.

Für die Gewinnung des Albuminanteiles der erfindungsgemäßen Lösung wird der Cohn IV - 1 + V-Niederschlag in Wasser gelöst. Für die anschließende Durchführung der Reinigung mittels Fraktionierung mit Ammonsulfat können literaturbekannte Methoden dienen. So ist es möglich, diese Fraktionierung mit Ammonsulfat allein durchzuführen, wie sie von G. Kander (1886) Arch. Exp. Pathol. Pharmakol. 20, 411 beschrieben ist. Es kann aber ebenso eine Ammonsulfatfraktionierung in Gegenwart von Kokosfettsäure dienen - siehe dazu z.B. AT-PS 208.507. Besonders bewährt hat sich dabei eine Vorgangsweise bei der der Cohn IV - 1 + V-Niederschlag zunächst in der 5 bis 6-fachen Menge an destilliertem Wasser gelöst und in der Lösung ein pH-Wert von 6,8 - 7,2 eingestellt wird. Nach Zugabe von Kokosfettsäure in einer Menge von etwa 3 g/l wird die Lösung rasch auf eine Temperatur von 58 - 63°C erhitzt und einige Zeit bei dieser Temperatur gerührt.

Bevor dann die erste Fällung mit Ammonsulfat durchgeführt wird, wird der pH-Wert mit Säure, z.B. Essigsäure, auf einen Wert von 4,8 - 5,1 gebracht. Es empfiehlt sich, mit Ammonsulfat bei diesem pH-Wert eine Vorfällung durchzuführen, die abzutrennen ist, bevor die Konzentration mit Ammonsulfat bei neutralem pH so erhöht wird, daß das Albumin gefällt wird. Zur Entfernung des Ammonsulfates wird die Lösung dann dialysiert. So gereinigte Albuminfraktionen werden dann zweckmäßigerweise stabilisiert, was auf übliche Weise erfolgen kann. Besonders gute Ergebnisse werden durch Zusatz von 0,16 mMol N-Acetyltryptophan/g Protein erhalten. Derart stabilisierte Lösungen können nach Verdünnen auf die gewünschte Proteinkonzentration von etwa 5 Gew.% und Zugabe der zur Erzeugung einer isotonen Lösung nötigen Menge an NaCl bei 60°C ± 0,5°C pasteurisiert werden. (Gellis et al, J. Klin. Invest. Vol 27, 239 -244, 1948), wobei z. B. eine Dauer von 10 Stunden gewählt werden kann.

Für die Bereitung der Globulinfraktion wird vom Cohn II/III-Niederschlag ausgegangen. Nach Aufnahme dieses nach Cohn erhaltenen Niederschlages in destilliertem Wasser wird das Äthanol durch Dialyse entfernt, bevor die erfindungsgemäße Wärmebehandlung bei 45 - 65$^{o}$C, vorzugsweise 52$^{o}$C, durchgeführt wird. Besonders gute Ergebnisse werden erhalten, wenn man vor der Wärmebehandlung durch eine isoelektrische Fällung bei einem pH-Wert von 5,2 - 5,4, vorzugsweise 5,3, die Euglobuline abtrennt. Ferner ist es vorteilhaft, anschließend durch eine absorptive Behandlung mit Bariumsulfat enzymatische Aktivitäten zu entfernen. Diese Behandlung wird zweckmäßig bei dem gleichen pH-Wert durchgeführt, wie die isoelektrische Fällung.

Zur Durchführung der Wärmebehandlung muß dann die Lösung neutralisiert werden.

Die Dauer der Wärmebehandlung beträgt bei 52$^{o}$C vorzugsweise etwa 30 Minuten, bei Wahl einer tieferen Temperatur entsprechend etwas länger. Dabei erfahren zuvor schon aggregierte Globuline eine weitere Aggregierung, sodaß sie in der Folge durch Zusatz von Ammonsulfat, vorzugsweise bis zu einer Konzentration von etwa 150 g/l unschwer ausgefällt und abgetrennt werden können.

Die zurückbleibende Lösung ist dann frei an unangenehm wirkenden Aggregaten. Aus ihr können die Globuline durch weitere Zugabe von Ammonsulfat, zweckmäßig bis zur Erzielung einer Konzentration in der Lösung von etwa 300 g/l gefällt werden. Die so erhaltene Globulinpaste kann dann durch Dialyse vom Ammonsulfat befreit werden. Sie wird ebenfalls mit Wasser auf eine Proteinkonzentration von etwa 5 Gew.% verdünnt und mit Natriumchlorid isoton gemacht.

Für die Endherstellung wird die nach dem Mischen resultierende Serumproteinlösung klärfiltriert, sterilisiert und in geeigneten Portionen in Flaschen abgefüllt. Sie ist dann bei Lagerung im Kühlschrank lange Zeit haltbar.

Die so erhaltenen Serumpräparate entsprechen in sämtlichen biologischen Testen, (Sterilität, Apyrogenität und Anomale Toxicität) und zeigen nach 4stündigem Erhitzen auf 57$^{o}$C keine Trübung oder Gelierung. Auf Grund der Untersuchung nach vorhandener antikomplementärer Aktivität kann gute intravenöse Verträglichkeit bescheinigt werden. (weniger als 20 CH50 von eingesetzten 100 CH50 werden gebunden)

Die Durchführung des erfindungsgemäßen Verfahrens soll anhand des folgenden Beispieles noch näher erläutert werden.

Beispiel

1. Albuminlösung:

8 kg Cohn IV - 1 + V-Niederschlag werden in 50 l destilliertem Wasser gelöst und durch Zugabe von konzentriertem Ammoniak wird ein pH-Wert von 7,1 eingestellt. Zu dieser Lösung gibt man 3 g/l Kokosfettsäure portionsweise zu und erhitzt schnell auf 60°C. Die Lösung wird dann 30 Minuten gerührt und über Nacht bei Raumtemperatur stehen gelassen.

Durch Zugabe von Essigsäure wird ein pH-Wert von 4,9 unter Einhalten einer Temperatur von unter 40°C eingestellt, worauf durch Zugabe von 300 g/l Ammonsulfat eine Vorfällung an Globulinen vorgenommen wird. Der erhaltene Niederschlag wird abgetrennt und verworfen. Das Filtrat wird mit konzentriertem Ammoniak auf einen pH-Wert von 7,25 eingestellt, und mit weiteren 200 g/l Ammonsulfat versetzt. Der so erhaltene Albuminniederschlag wird abfiltriert, mit destilliertem Wasser aufgeschlämmt und bei 4°C 4 Tage lang gegen demineralisiertes Wasser dialysiert. Nach Abschluß der Dialyse wird durch Verdünnen ein Proteingehalt von 5 Gew.% eingestellt und die Lösung durch Zugabe von 140 mMol Natriumchlorid/l isoton gemacht. Zur Stabilisierung wird sie mit 0,16 mMol N-Acetyltryptophan/g Protein versetzt, auf einen pH-Wert von 7 eingestellt, durch Klär- und Sterilfiltration gereinigt und 10 Stunden durch Erhitzen auf 60°C pasteurisiert.

2. Globulinlösung:

4 kg Cohn II + III Paste werden mit der gleichen Menge destilliertem Wasser gemischt und 4 Tage bei 4°C gegen demineralisiertes Wasser dialysiert. Anschließend wird die Lösung mit der 10-fachen Menge an destilliertem Wasser verdünnt, mit 20 %iger Essigsäure auf einen pH-Wert von 5,35 eingestellt, 2 Stunden gerührt und über Nacht stehen gelassen. Nach Zugabe von Filtercel (R) als Filterhilfsmittel wird der beim Stehen gebildete Niederschlag abfiltriert und verworfen. Das Filtrat wird unter Rühren mit 30 g/l Bariumsulfat versetzt, worauf noch 1 Stunde weitergerührt und wiederum einige Stunden stehen gelassen wird. Unter nochmaliger Zugabe von Filterhilfsmittel wird das Bariumsulfat abfiltriert und das Filtrat wird anschließend mit konzentriertem Ammoniak auf einen pH-Wert von 7,0 eingestellt. Die so erhaltene neutrale Lösung wird dann unter Rühren 30 Minuten lang auf $52^{\circ}C$ erwärmt, auf $25^{\circ}C$ abgekühlt und zur Fällung der aggregierten Proteine mit 150 g/l Ammonsulfat versetzt. Nach zweistündigem Rühren wird die Mischung einige Stunden bei Raumtemperatur stehengelassen. Der so gebildete Niederschlag wird abfiltriert und das Filtrat nach Klärfiltration unter Rühren mit weiteren 150 g/l Ammonsulfat versetzt. Nach zweistündigem Rühren wird der so erhaltene Niederschlag der gereinigten Globuline abgetrennt, mit destilliertem Wasser aufgeschlämmt, 4 Tage lang bei $4^{\circ}C$ gegen demineralisiertes Wasser dialysiert, auf eine Proteinkonzentration von 5 Gew.% verdünnt und durch Zugabe von etwa 140 mMol Natriumchlorid pro Liter isoton gemacht.

3. Mischung der Lösungen:

4 Volumsteile der nach 1) erhaltenen Albuminlösung werden mit einem Volumsteil der nach 2) erhaltenen Globulinlösung gemischt, anschließend klar- und sterilfiltriert. Man erhält 16,2 Liter Lösung, die in entsprechende Einheiten abgefüllt wird.

Die so erhaltene Serumproteinlösung ist gebrauchsfertig.

Ihr Proteingehalt beträgt 4,9 Gew.%.

Dieser setzt sich zusammen aus 65,7 Gew.% Albumin, 3,6 Gew.% alpha-Globulin, 14 Gew.% beta-Globulin und 15,9 Gew.% gamma-Globulin.

Der Gehalt an den einzelnen Immunglobulinfraktionen beträgt:

| | |
|---|---|
| Ig G . | 4,8 g/l |
| Ig A | 2,1 g/l |
| Ig M | 0,9 g/l |

Die Antikomplementäraktivität war gegenüber natürlichem Serum unverändert.

Patentansprüche

1. Verfahren zur Herstellung einer Serumproteinlösung mit gegenüber natürlichem Blutserum unveränderter Antikomplementäraktivität, die für die intravenöse Verabreichung als Plasmasubstitut geeignet ist, durch Fraktionierung von Humanplasma oder Humanserum mittels Äthanol nach Cohn, sowie Mischen und Auflösen von dabei erhaltenen, gegebenenfalls weiter gereinigten Albumin- und Globulinfraktionen in isotoner Kochsalzlösung, dadurch gekennzeichnet, daß sowohl die bei der Cohn-Fraktionierung erhaltene, als Fraktion IV - 1 und V bezeichnete Albuminfraktion, als auch die als Cohn II und III-Niederschlag bezeichnete Globulinfraktion getrennt einer Reinigung mittels Ammonsulfatfällung unterzogen werden, wobei die Globulinfraktion vor der Fällung mit Ammonsulfat in Form einer wäßrigen Lösung bei neutralem pH durch eine Wärmebehandlung bei 45 - 65°C einer gezielten Aggregatbildung zuvor aggregierter Anteile sowie die gereinigte Albuminfraktion einer Pasteurisierung unterworfen wird, bevor sie mittels Wasser und Kochsalz in zwei getrennte isotone Lösungen gleichen oder annähernd gleichen Proteingehaltes übergeführt, im gewünschten Verhältnis gemischt und in üblicher Weise durch Klär- und Sterilfiltration in haltbare Form übergeführt werden.

2. Verfahren nach Anspruch 1, dadurch gekennzeichnet, daß sowohl die Albuminlösung als auch die Globulinlösung auf einen Gehalt von etwa 4,75 bis 5,25 Gew.% Protein gebracht werden und einen Kochsalzgehalt von etwa 130 - 160 mMol NaCl pro Liter aufweisen.

3. Verfahren nach den Ansprüchen 1 und 2, dadurch gekennzeichnet, daß die Albuminlösung mit der Globulinlösung etwa im Volumsverhältnis von 4 : 1 gemischt wird.

4. Verfahren nach den Ansprüchen 1 bis 3, dadurch gekennzeichnet, daß der Cohn II und III-Niederschlag nach dem Auflösen aber vor der Wärmebehandlung zur Abtrennung der Euglobuline einer isoelektrischen Fällung bei einem pH-Wert von 5,2 bis 5,4 unterzogen, der erhaltene Niederschlag abfiltriert und das Filtrat nach Neutralisation der Wärmebehandlung zugeführt wird.

5. Verfahren nach Anspruch 4, dadurch gekennzeichnet, daß das Filtrat der Euglobulinfällung vor der Wärmebehandlung durch Absorption mittels Bariumsulfat von enzymatischen Aktivitäten befreit wird.

6. Verfahren nach den Ansprüchen 1 bis 5, dadurch gekennzeichnet, daß die Dauer der Wärmebehandlung bei 52°C etwa 30 Minuten beträgt.

7. Verfahren nach den Ansprüchen 1 bis 6, dadurch gekennzeichnet, daß aus der aus der Wärmebehandlung resultierenden Lösung die dabei gebildeten Aggregate durch Fällung mittels Zugabe von Ammonsulfat bis zu einer Konzentration von etwa 150 g/Liter ausgefällt, diese anschließend abgetrennt werden und die gereinigten Globuline durch Erhöhung der Ammonsulfatkonzentration auf eine Konzentration von etwa 300 g/l als Niederschlag gewonnen werden.

O.Z. 742
26. 9.1984